# EUROPEAN PATENT SPECIFICATION

(11) **EP 1 547 617 B1**
(45) Date of publication and mention of the grant of the patent: **28.04.2010**
(21) Application number: 03792716.7
(22) Date of filing: 19.08.2003
(51) Int. Cl.: A61K 45/00, A61K 31/16, A61K 31/167, A61K 31/19, A61K 31/4045, A61K 31/4406, A61K 38/55, A61P 19/02, A61P 43/00

(54) **ARTHRODIAL CARTILAGE EXTRACELLULAR MATRIX DEGRADATION INHIBITOR**
HEMMER DES ABBAUS DER EXTRAZELLULÄREN MATRIX VON ARTHRODIA-KNORPEL
INHIBITEUR DE DEGRADATION DE LA MATRICE EXTRACELLULAIRE DU CARTILAGE ARTHRODIAL

(30) Priority: 20.08.2002 JP 2002239203
(43) Date of publication of application: 29.06.2005
(73) Proprietor: Astellas Pharma Inc., Chuo-ku, Tokyo (JP)
(72) Inventor: YAMAJI, Noboru, Tokyo 103-8411 (JP); SHINDOU, Nobuaki, Tokyo 103-8411 (JP); TERADA, Yoh, Tokyo 103-8411 (JP)
(74) Representative: Bates, Philip Ian
(86) International application number: PCT/JP2003/010460
(87) International publication number: WO 2004/017996

(56) References cited:
- WO-A-03/075929
- WO-A-03/087057
- WO-A1-02/06307
- WO-A1-95/13289
- WO-A2-02/22577
- WO-A2-02/055017
- JP-A- 2001 348 340
- JP-A- 2001 354 694
- NAOKI ISHIGURO: 'Mansei kansetsu rheumatism to saibogai matrix hakai' IGAKU NO AYUMI vol. 177, no. 1, 1996, pages 81 - 85, XP002975645
- SAITO A. ET AL.: 'A synthetic inhibitor of histone deacetylase, MS-27-275m, with marked in vivo antitumor activity against human tumors' PROC. NATL. ACAD. SCI. USA vol. 96, no. 8, 1999, pages 4592 - 4597, XP002158228
- NAKAJIMA H. ET AL.: 'FR901228, a potent antitumor antibiotic, is a novel histone deacetylase inhibitor' EXP. CELL. RES. vol. 241, no. 1, 1998, pages 126 - 133, XP002928826
- RICHON V.M. ET AL.: 'A class of hybrid polar inducers of transformed cell differentiation inhibits histone deacetylase' PROC. NATL. ACAD. SCI. USA vol. 95, no. 6, 1998, pages 3003 - 3007, XP001120542
- HAN J.-W. ET AL.: 'Apicidin, a histone deacetylase inhibitor, inhibits proliferation of tumor cells via induction of p21WAF1/Cip1 and gelsolin' CANCER RES. vol. 60, no. 21, 2000, pages 6068 - 6074, XP002960288
- WARRELL R.P. JR. ET AL.: 'Therapeutic targeting of transcription in acute promyelocytic leukemia by use of an inhibitor of histone deacetylase' J. NATL. CANCER INST. vol. 90, no. 21, 1998, pages 1621 - 1625, XP002928827
- JOHNSTONE R.W. ET AL.: 'Histone-deacetylase inhibitors: novel drugs for the treatment of cancer' NAT. REV. DRUG DISCOV. vol. 1, no. 4, April 2002, pages 287 - 299, XP002975646
- GOTTLICHER M. ET AL.: 'Valproic acid defines a novel class of HDAC inhibitors inducing differentiation of transformed cells' EMBO J. vol. 20, no. 24, 2001, pages 6969 - 6978, XP002975647

## Description

### Technical Field

The present invention relates to an agent for inhibiting articular cartilage extracellular matrix degradation effective as an agent for treating joint diseases such as arthrosteitis, rheumatic arthritis, and osteoarthritis.

### Background Art

A DNA in a cell nucleus forms a chromatin structure in which a nucleosome is the fundamental unit. A nucleosome is a structure in which a core histone (an octamer composed of each two molecules of histones H2A, H2B, H3, and H4) and a DNA are entwined each other and positively charged lysine residues present at an N-terminal of the histones form a stable state in a charge together with a negatively charged DNA, whereby the nucleosome is present in a highly folded state (Wolffe, A.P. et al., Cell, 84, 817-819, 1996). In order to enable the occurrence of transcription in a nucleus, it is necessary to make the structure in a loose state to thereby enable various transcription factors to contact with the DNA. The relationship between acetylation of histones and activation of transcription has been known such that histones in a gene region where transcription is suppressed is less acetylated and histones in a gene region where transcription actively occurs is highly acetylated (Hebbes, T.R. et al., EMBO J., 7, 1395-1402, 1988, Grunstein, M. et al., Nature, 389, 349-352, 1997). It is considered that when the lysine residues of a histones in a nucleosome are acetylated, the positive charge is neutralized and the nucleosome structure is loosened, so that it becomes possible for various transcription factors to contact with the DNA and thus occurrence of transcription is facilitated (Hong, L. et al., J. Biol. Chem., 268, 305-314, 1993).

It is known that acetylation of histones is regulated by the balance between histone acetylases (histone Acetyltransferases; HATs) and histone deacetylases (Histone Deacetylases; HDACs). Recently, some HATs and HDACs have been identified and the importance in transcriptional regulation thereof has been reported (Ogryzko, V.V. et al., Cell, 87, 953-959, 1996, Brown, C.E. et al., Trends Biochem. Sci., 25(1), 15-19, 2000; Grozinger, C.M. et al., Proc. Natl. Acad. USA, 96, 4868-4873, 1999).

On the other hand, it has been known that butyric acid which has various activities such as cell cycle arrest, morphological normalization of transformed cells, and induction of differentiation accumulates highly acetylated histones in a cell and thus has an HDAC inhibitory activity (Counsens, L.S. et al., J. Biol. Chem., 254, 1716-1723, 1979). Moreover, Trichostatin A (TSA) which is a microbial metabolite has been known to exhibit cell cycle arrest and induction of differentiation (Yoshida, M. et al., Cancer Res., 47, 3688-3691, 1987, Yoshida, M. et al., Exp. Cell Res., 177, 122-131, 1988). Based on the investigation in which highly acetylated histones are accumulated in a cell and a partially purified HDAC is used, it has been revealed that TSA is a potent HDAC inhibitor (Yoshida, M. et al., J. Biol. Chem., 265, 17174-17179, 1990).

Studies have been carried out on the other HDAC inhibitors. It has been known that Trapoxin which is a microbial metabolite has an activity of inhibiting cell proliferation and normalizing the morphology of v-sis transformed cells (Itazaki, H. et al., J. antibiotics, 43(12), 1524-1534, 1990), and later, it has been revealed that the substance is an HDAC inhibitor (Kijima, M. et al., J. Biol. Chem., 268, 22429-22435, 1993). Since the inhibition mode is irreversible, molecular cloning of a human HDAC gene which binds to Trapoxin used as a molecular probe has been reported (Taunton, J. et al., Science, 272, 408-411, 1996). In addition, compounds such as Depudecin (Kwon, H.J. et al., Proc. Natl. Acad. Sci. USA, 95, 3356-3361, 1998), Phenylbutyrate (Warrell, R.P.Jr. et al., J. Natl. Cancer Inst., 90(21), 1621-1625, 1998), Pivaloyloxymethyl butyrate (Aviram, A. et al., Int. J. Cancer, 56, 906-909, 1994), MS-27-275 (Saito, A. et al., Proc. Natl. Acad. Sci. USA, 96, 4592-4597, 1999), CI-994 (Howard, C.T et al., Proc. Am. Assoc. Cancer Res. abst #2886, 2002), SAHA (Richon, V.M. et al., Proc. Natl. Acad. Sci. USA, 95, 3003-3007, 1998), CHAP (cyclic hydroxamic acid-containing peptide) (Furumai, R. et al., Proc. Natl. Acad. Sci. USA, 98, 87-92, 2001), Valproic acid (Gottlicher, M. EMBO J., 20, 6969-78, 2001), NVP-LAQ824 (Perez, L.B. et al., Proc. Am. Assoc. Cancer Res. abst #3671, 2002, WO02/22577), and Apicidin (Cancer Res., 60, 6068-6074, 2000) have been reported to have an HDAC inhibitory activity.

Moreover, recently, it has been reported that some depsipeptide derivatives have a good HDAC inhibitory activity. For example, FK228 (Nakajima, H. et al., Exp. Cell Res., 241, 126-133, 1998) and the depsipeptide compound represented by the following formula (I) (International Publication WO02/06307 pamphlet: Patent Reference 1), the depsipeptide compound represented by the following general formula (II) (JP-A-2001-348340), the depsipeptide compound represented by the following general formula (IIa) (JP-A-2001-354694), and the like have been reported. Hereinafter, the depsipeptide compound represented by the formula (I) is referred to as a FK228 reduced form, the substance where R is an isopropyl group in the general formula (II) as Compound A, the substance where R is a sec-butyl group as Compound B, the substance where R is an isobutyl group as Compound C, and the depsipeptide compounds represented by the general formula (IIa) as reduced forms of Compounds A to C: 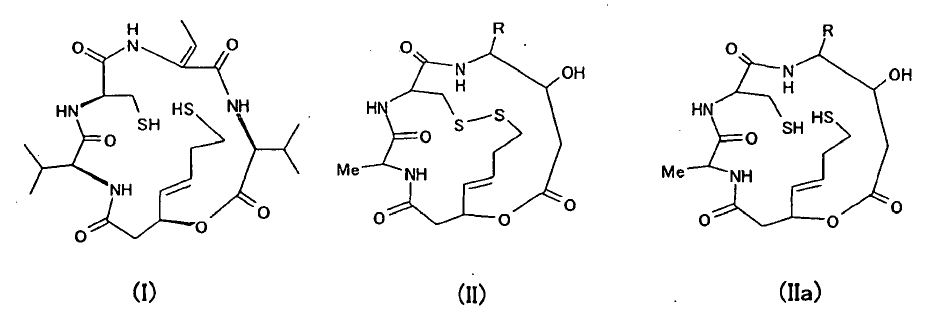 wherein R represents an isopropyl group, a sec-butyl group, or an isobutyl group.

Since the HDAC inhibitors exhibit such as cell cycle arrest, morphological normalization of transformed cells, induction of differentiation, induction of apoptosis and angiogenesis inhibitory activity, an effect as an antitumor agent has been expected (cf. Non-Patent References 1 and 2). Additionally, various applications, e.g., an agent for the treatment and improvement of cell-proliferating diseases such as infectious diseases, autoimmune diseases, and skin diseases (cf. Non-Patent Reference 3), and an agent for the prevention and treatment of progressive neurodegenerative diseases such as Huntington's disease (cf. Non-Patent Reference 4), and furthermore promotion of efficiency of vector transduction in genetic therapy (cf. Non-Patent Reference 5), and enhancement of expression of transduced genes (cf. Non-Patent Reference 6) have been attempted.

However, there has been no specific report which shows the relationship between HDAC and the articular cartilage extracellular matrix and also, there has been no report which indicates that an HDAC inhibitor inhibits degradation and degeneration of articular cartilage extracellular matrix components and is effective for the prevention and treatment of joint diseases involving degradation and degeneration of the articular cartilage extracellular matrix components.

Additionally, in the patent specification (Patent Reference 1) for the FK228 reduced form, a number of diseases are cited for the reason that the FK228 reduced form is effective for diseases induced by abnormal gene expression by its HDAC-inhibitory activity. Therein, although rheumatic arthritis and osteoarthritis are cited, any specific effect is not described and the basis which indicates a therapeutic effect is not shown. Moreover, International Publication WO02/055017 pamphlet (Patent Reference 2) describes that an HDAC inhibitor can be used for the treatment of autoimmune diseases including rheumatic arthritis on the basis of the fact that the HDAC-inhibitor exhibits an effect of normalizing abnormal expression of immune-related genes in T cells collected from patients of systemic lupus erythematosus. However, there is no disclosure of specific effect for rheumatic arthritis and also no description which indicates the HDAC inhibitor inhibits degradation and degeneration of articular cartilage extracellular matrix.

The joint diseases such as arthrosteitis, rheumatic arthritis (RA), and osteoarthritis (OA) are diseases in which damage and degeneration of articular cartilage is a main lesion. Among the joint diseases, the disease with which the largest number of patients suffer from is OA. However, in the current method for treatment, analgesic anti-inflammatory agents or hyaluronic acid preparations are only used as symptomatic treatment for the purpose of alleviating pain involved in cartilage degeneration and subchondral bone destruction. Therefore, it cannot be said that a sufficient therapeutic effect is achieved.

The joint diseases are induced by various causes such as cracks on cartilage surface due to external injury, autoimmune disorder and disorder of matrix metalloprotease, and at the initial stage, degradation and degeneration of an extracellular matrix in articular cartilage are commonly observed (cf. Non-Patent References 7 and 8). The extracellular matrix is mainly composed of type II collagen and aggrecan which is a cartilage-specific proteoglycan, and abnormality of either of them causes destruction of the extracellular matrix to result in destruction of cartilage tissue. Furthermore, in RA, the destruction of cartilage tissue causes exposure, destruction, and degeneration of subchondral bone tissue to invite disorder of articular function. On the other hand, in OA, the destruction causes osteophyte formation and bone sclerosis due to proliferation of subchondral bone to invite joint degeneration (cf. Non-Patent Reference 8). Therefore, from long ago, it is considered that regulation of degradation and degeneration of the extracellular matrix which are common to these joint diseases may lead to treatment of joint diseases and thus identification of proteases (collagenase and aggrecanases) which take charge of the degradation, search for their inhibitors, and attempts to develop them as medicaments have been intensively carried out (cf. above Non-Patent References 7, 9, and 10). However, up to now, any agent for articular disease, which regulates degradation and degeneration of the articular cartilage extracellular matrix is not placed on the market (cf. Non-Patent References 11 and 12).

It has been still highly desired to develop a medicament which satisfactorily inhibits degradation of the articular cartilage extracellular matrix.
[Non-Patent reference 1] Marks, P.A. et al., "Journal of the National Cancer Institute", 2000, Vol. 92, p.1210-1216
[Non-Patent reference 2] Kim, M.S. et al., "Nature Medicine", 2001, Vol. 7, p.437-443
[Non-Patent reference 3] Darkin-Rattray, S.J. et al., "Proceedings of the National Academy of Science of United State of America", 1996, Vol. 93, p.13143-13147
[Non-Patent reference 4] Steffan, J.S. et al., "Nature", 2001, Vol. 413, p. 739-743
[Non-Patent reference 5] Dion, L.D. et al., "Virology", 1997, Vol. 231, p. 201-209
[Non-Patent reference 6] Chen, W.Y. et al., "Proceedings of the National Academy of Sciences of United States of America", 1997, Vol. 94, p.5798-5803
[Non-Patent reference 7] Ishiguro, N, "Igaku no Ayumi (extracellular matrix) additional volume", Ishiyaku Shuppan K.K., February 25, 1997, p.81-85
[Non-Patent reference 8] Ozaki, S et al., "Shitteokitai Hone/Kansetu Shikkan no Aratana Shinryo", Shinko Ko-eki K.K. Ishoshuppan-bu, November 20, 2001, p.46-95
[Non-Patent reference 9] Lohmander, LS. et al., "Arthritis and Rheumatism", 1993, Vol. 36, No. 9, p.1214-22
[Non-Patent reference 10] Malfait, A.M. et al., "Journal of Biological Chemistry", 2002, Vol. 277, No. 25, p.22201-8
[Non-Patent reference 11] Close, D.R., "Annals of the Rheumatic Diseases", 2001, Vol. 60, No. 3, p.62-67
[Non-Patent reference 12] Arner, E.C. et al., "Current Opinion in Pharmacology", 2002, Vol. 2, p. 322-329
[Patent reference 1] International Publication WO02/06307 pamphlet
[Patent reference 2] International Publication WO02/055017 pamphlet

### Disclosure of the Invention

As a result of the intensive studies on the activities of HDAC-inhibiting compounds, the inventors of the present invention found that many HDAC-inhibiting compounds which are completely different in structure have a good inhibitory activity of articular cartilage extracellular matrix degradation, and thus they have accomplished the present invention.

Namely, the present invention relates to an agent for inhibiting articular cartilage extracellular matrix degradation comprising an HDAC-inhibiting compound as an active ingredient. Particularly, the present invention relates to an agent for the prevention and treatment of arthrosteitis, rheumatic arthritis, osteoarthritis, and the like which degradation and degeneration of the articular cartilage extracellular matrix relates to.

Also, the present invention relates to an agent for the prevention and treatment of articular cartilage extracellular matrix degradation in arthrosteitis, rheumatic arthritis, osteoarthritis and the like, comprising an HDAC-inhibiting compound as an active ingredient.

Moreover, the present invention relates to a use of an HDAC-inhibiting compound for producing a medicament for inhibiting articular cartilage extracellular matrix degradation.

Furthermore, the present invention relates to a method for preventing or treating a disease caused by articular cartilage extracellular matrix degradation, which comprises administrating a therapeutically effective amount of an HDAC-inhibiting compound to a patient.

The present invention reveals a possibility that an HDAC-inhibiting compound, which relates to transcriptional regulation, unexpectedly suppresses degradation and degeneration of the articular extracellular matrix itself which is a main lesion of arthrosteitis and can bear a central role in treatment of arthrosteitis. Thus, the invention is a really remarkable invention.

The present invention is described below in detail.

The HDAC-inhibiting compound for use in the invention is a compound inhibiting HDAC or a salt thereof. Specifically, it includes such as FK228 and its reduced form, depsipeptide compounds (Compounds A, B, and C) and their reduced forms, MS-27-275, Trichostatin A, NVP-LAQ824, SAHA, Apicidin, butyric acid and its derivatives (Phenylbutyrate, Pivaloyloxymethyl butyrate, Valproic acid, etc.), CI-994, Depudecin, Trapoxin and CHAP, which are known to be HDAC-inhibiting compounds. These HDAC-inhibiting compounds are commercially available or obtainable using methods known by references. Preferred ones are FK228 and its reduced form, depsipeptide compounds (Compounds A, B, and C) and their reduced forms, MS-27-275, Trichostatin A, NVP-LAQ824, SAHA, Apicidin, Phenylbutyrate, and Valproic acid, and more preferred ones are FK228 and its reduced form, depsipeptide compounds (Compounds A, B, and C) and their reduced forms, MS-27-275, Trichostatin A, NVP-LAQ824, SAHA, and Apicidin. Additionally, derivatives of these compounds having a similar activity are also suitable as the HDAC-inhibiting compounds of the present invention.

The HDAC inhibitory activity can be easily measured in accordance with known general methods, e.g., the method described in Yoshida, M. et al., J. Biol. Chem., 265, 17174-17179. Specifically, the measurement can be conducted, using [³H]acetylhistone and a histone deacetylase fraction prepared by the method described in the above reference, adding a test compound to a reaction solution containing [³H]acetylhistone and DTT, mixing the histone deacetylase fraction after 1 hour of pre-incubation at room temperature, reacting them for 2 hours at room temperature, adding 1M hydrochloric acid and ethyl acetate thereto, and then measuring radioactivity in the ethyl acetate layer separated by centrifugation by a scintillation counter.

The values of the HDAC inhibitory activity of representative HDAC-inhibiting compounds are shown together with the names of cited references. In this connection, with regard to Compound A, a result measured in accordance with the above method is shown.
- FK228: 1.1 nM (IC₅₀ value; Exp. Cell Res., 241, 126-133, 1998)
- Compound A: about 85% inhibition at 30 nM
- Ms-27-275: 2 µM (IC₅₀ value; Proc. Natl. Acad. Sci. USA, 96, 4592-4597, 1999) .
- Butyric acid (Na salt): 280 µM (IC₅₀ value; Exp. Cell Res., 241, 126-133, 1998)
- Butyrates (butyric acid, Phenylbutyrate, etc.): mM order (Nat. Rev. Drug Discov., 2002 1, 287-299, 2002)
- Valproic acid: mM order (Nat. Rev. Drug Discov., 2002 1, 287-299, 2002)
- Trichostatin A: 2.1 nM (IC₅₀ value; Exp. Cell Res., 241, 126-133, 1998)
- NVP-LAQ824: 0.03 µM (IC₅₀ value; Blood First Edition Paper, prepublished online, June 19, 2003) .
- SAHA: 10-20 nM (IC₅₀ value; Proc. Natl. Acad. Sci. USA, 95, 3003-3007, 1998)
- Apicidin: 5 nM (IC₅₀ value; Cancer Res., 60, 6068-6074, 2000)

Other preferable HDAC-inhibiting compounds of the present invention are compounds which have an HDAC inhibitory activity (IC₅₀ value) measured by the method of Yoshida *et al*. of 100 µM or less, more preferably 10 µM or less, further preferably 1 µM or less.

A formulation method and administration method of the agent for inhibiting articular cartilage extracellular matrix degradation of the present invention is described below in detail.

A pharmaceutical composition comprising one, or two or more types of the HDAC-inhibiting compound as the active ingredient is prepared into tablets, powders, fine granules, granules, capsules, pills, liquids, injections, suppositories, ointments, patches, and the like, using carriers and excipients generally used for formulation, and other additives, and is orally or parenterally administered.

The clinical dose of the HDAC-inhibiting compound to humans is appropriately determined, depending on the kind of the HDAC-inhibiting compound. As usual, the appropriate dose is approximately from 0.001 to 500 mg, preferably from 0.01 to 300 mg per day in the case of oral administration, which is administered once a day or by dividing into 2 to 4 times per day. Also, in the case of parenteral administration, e.g., intra-articular, intramuscular, subcutaneous, or intravenous administration, it is appropriate approximately from 0.0001 to 100 mg, preferably 0.001 to 10 mg per dose. The administration frequency and dose are appropriately determined in depending on symptom, age, sex, and the like according to individual cases.

As the solid composition for oral administration of the present invention, tablets, powders, granules, and the like are used. For such a solid composition, one or more active substances are mixed with at least one inactive diluent, for example lactose, mannitol, glucose, hydroxypropyl cellulose, microcrystalline cellulose, starch, polyvinylpyrrolidone, magnesium aluminometasilicate, and the like. According to usual methods, the composition may contain additives other than inactive diluents, for example lubricants such as magnesium stearate, disintegrators such as cellulose calcium glycolate, and further stabilizers or dissolution-auxiliary agents. If necessary, the tablets or pills may be coated with films of sugar coating such as sucrose, gelatin, hydroxypropyl cellulose, hydroxypropylmethyl and cellulose phthalate, or coating of enteric or gastric compounds.

The liquid composition for oral administration includes pharmaceutically acceptable emulsions, solutions, suspensions, syrups, and elixirs, and the like and also includes inactive diluents generally used, for example purified water and ethyl alcohol. The composition may contain auxiliary agents such as dissolution-auxiliary agents, moisturizers and suspending agents, sweeteners, flavoring agents, aromatics and antiseptics in addition to inactive diluents.

The injections for parenteral administration includes aseptic and aqueous or non-aqueous solutions, suspensions and emulsions. The diluents for aqueous solutions and suspensions include, for example, distilled water for injections and physiological saline. The diluents for non-aqueous solutions and suspensions include, for example, propylene glycol, polyethylene glycol, vegetable oils such as olive oil, alcohols such as ethyl alcohol, polysorbate 80 (trade name), and the like. Such composition may further contain additives such as isotonic agents, antiseptics, moisturizers, emulsifiers, dispersants, stabilizers (e.g., lactose), and dissolution-auxiliary agents. These are sterilized by filtration through, for example, bacteria-retaining filter, blending with germicides, or irradiation. These can be prepared into aseptic solid compositions and the compositions can be used, after dissolution in aseptic water or aseptic solvents for injections prior to use.

When the compound for use in the medicament of the present invention has a low solubility, a solubilization treatment may be carried out. As the solubilization treatment, known methods applicable to pharmaceutical preparations, for example, a method of adding a surfactant and a method of forming a solid dispersed form of a drug with a solubilizing agent such as a polymer (water-soluble polymer or enteric polymer) may be cited.

### Best Mode for Carrying Out the Invention

The articular cartilage extracellular matrix degradation inhibitory activity of HDAC-inhibiting compounds which are active ingredients of the present invention is described below in detail.

### Example 1: Proteoglycan (PG) destruction inhibitory activity in rabbit cartilage primary culture cell

### (stimulation with retinoic acid)

### (Test method)

After a rabbit (Japanese white race, male, 1.0 to 1.5 kg) was killed under excessive anesthesia, the knee joint was taken out and the cartilage layer on the articular surface was abraded and cut finely. Furthermore, after 1 hour of treatment with trypsin-EDTA (0.25%-1 mM; manufactured by GIBCO-BRL) at 37°C, the cut layer was centrifuged at 1500 rpm for 5 minutes and precipitated cells were washed with Dulbecco's modified eagle medium (DMEM, manufactured by GIBCO-BRL). Subsequently, after the layer was treated with collagenase A (0.15%; manufactured by Boehringer Mannheim)/DMEM at 37°C for 3 to 12 hours, a fraction passed through a nylon mesh filter (100 µm, manufactured by Falcon) was centrifuged at 1,500 rpm for 5 minutes to precipitate cartilage cells. After washing the cells with DMEM/10% FBS medium, the cells were suspended in DMEM/10% FBS medium so as to be 2×10⁵ cells/ml, and were inoculated to a 96-well plate coated with type I collagen (manufactured by Asahi Technoglass Corporation) at an amount of 200 µl/well. After 3 days, the medium was replaced by 200 µl of DMEM/10% FBS medium containing 50 µg/ml ascorbic acid (hereinafter referred to as ascorbic acid medium) and two days of culture was further repeated twice. Thereafter, the rabbit knee articular cartilage primary culture cells were cultured in 200 µl of the ascorbic acid medium containing Na₂³⁵SO₄ of a final concentration of 10 µCi/ml for 3 days to achieve labeling, followed by washing with 200 µl of the ascorbic acid medium three times and 1 day of culture in 200 µl of the ascorbic medium. The culture was stimulated with all-trans retinoic acid (manufactured by Sigma) of a final concentration of 1 µM. A culture supernatant after 48 hours was recovered in an each amount of 20 µl and radioactivity was measured using Topcount (manufactured by Packard). The test compound was added simultaneously with the start of the stimulation and a PG degradation inhibitory activity was calculated as percentage where a group to which retinoic acid was not added was defined as 100% and a group to which retinoic acid was added was defined as 0%.

### (Test Compounds)

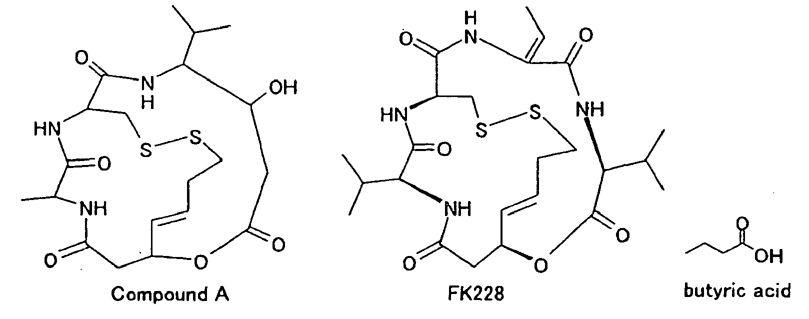

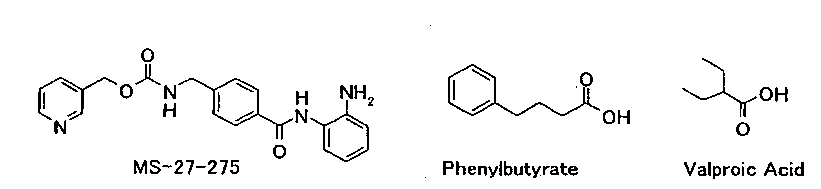

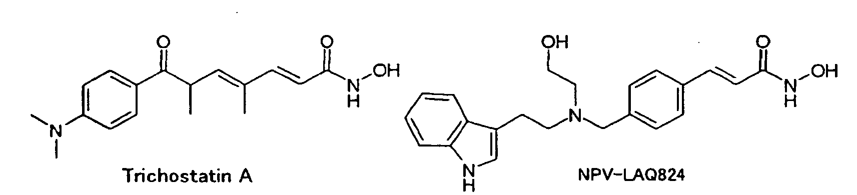

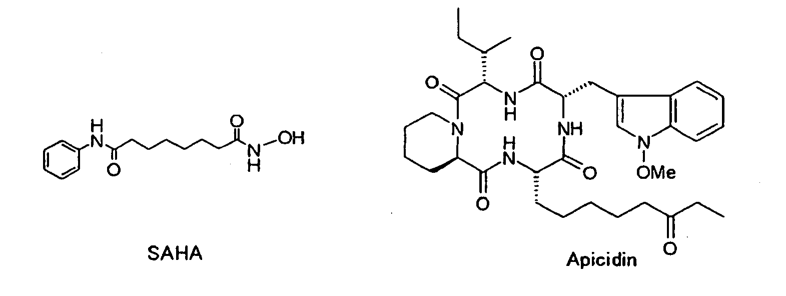

### (Results)

The results are shown in the following Table 1. It was revealed that each HDAC-inhibiting compound inhibited PG degradation in a concentration-dependent manner in the concentration range where its HDAC inhibition was shown, regardless of the structure.

**Table 1**

| Test compound | Concentration | PG degradation inhibition (%) |
|---|---|---|
| Compound A | 1000 nM | 98 |
| | 100 nM | 93 |
| | 10 nM | 51 |
| | 1 nM | 20 |
| FK228 | 100 nM | 97 |
| | 10 nM | 39 |
| MS-27-275 | 10 iM | 87 |
| | 1 iM | 52 |
| Butyric acid (Na salt) | 3 mM | 93 |
| | 0.3 mM | 27 |
| Phenylbutyrate | 3 mM | 66 |
| | 1 mM | 14 |
| | 0.3 mM | -2 |
| Valproic acid (Na salt) | 3 mM | 79 |
| | 1 mM | 46 |
| | 0.3 mM | 49 |
| Trichostatin A | 1000 nM | 58 |
| | 100 nM | 40 |
| NVP-LAQ824 | 300 nM | 90 |
| | 30 nM | 26 |
| SAHA | 3000 nM | 71 |
| | 300 nM | 13 |
| Apicidin | 300 nM | 92 |
| | 30 nM | 25 |

### Example 2: PG destruction inhibitory activity in rabbit cartilage primary culture cell (stimulation with IL-1)

### (Test method)

In the same manner as in Example 1, rabbit cartilage primary culture cells were prepared. They were stimulated with human IL-1β (manufactured by R&D System) of a final concentration of 10 ng/ml. A culture supernatant after 48 hours was recovered in an each amount of 20 µl and radioactivity was measured using Topcount (manufactured by Packard). The test compound was added simultaneously with the start of the stimulation and a PG degradation inhibitory activity was calculated as percentage where a group to which IL-1 was not added was defined as 100% and a group to which IL-1 was added was defined as 0%.

### (Results)

The results are shown in the following Table 2. It was revealed that each HDAC-inhibiting compound which are test compounds inhibited PG degradation in the concentration range where the HDAC inhibition was shown.

**Table 2**

| Test compound | Concentration | PG degradation inhibition (%) |
|---|---|---|
| Compound A | 30 nM | 61 |
| | 3 nM | 39 |
| | 0.3 nM | 6 |
| FK228 | 30 nM | 97 |
| MS-27-275 | 30 iM | 95 |
| Butyric acid (Na salt) | 5 mM | 78 |

The test methods of the above Examples 1 and 2 are methods conventionally and widely used for screening of an agent for treating articular disease as convenient and simple evaluation methods for evaluating an activity of a test compound toward articular cartilage, particularly an activity toward degradation and degeneration of extracellular matrix (Spirito S. et al., Agents Actions; 39, C160-2, 1993). Actually, matrix metalloprotease inhibitors which has an activity of suppressing PG degradation by retinoic acid, IL-1, or the like *in vitro* (Lawrence J. et al., Arch Biochem Biophys., 344(2), 404-12, 1997) was confirmed to suppress degradation and degeneration of an extracellular matrix even in a arthrosteitis-model mouse (Ann Rheum Dis., 54(8), 662-9, 1995, J Exp Med. 182(2), 449-57, 1995, Adv Dent Res., 12(2), 82-5, 1998 and Inflamm Res., 49(4), 144-6, 2000). Accordingly, the HDAC-inhibiting compounds which were confirmed to have excellent effects in the present evaluation systems and are active ingredients of the present invention are useful as agents for suppressing degradation and degeneration of the articular cartilage extracellular matrix. In particular, the HDAC-inhibiting compounds which are active ingredients of the present invention have an activity of satisfactorily suppressing degradation and degeneration of the articular cartilage extracellular matrix against both of IL-1 and retinoic acid which are representative stimulating substances inducing degradation and degeneration of the cartilage extracellular matrix, and hence the compounds are useful as agents for suppressing a cartilage extracellular matrix, regardless of the kind of stimulating substances.

### Industrial Applicability

Since the HDAC-inhibiting compound which is an active ingredient of the medicament of the present invention satisfactorily inhibits degradation of the articular cartilage extracellular matrix as shown in the above Examples, the medicament of the present invention is particularly useful as a preventing or treating agent for arthrosteitis, rheumatic arthritis, osteoarthritis, and the like involving degradation and degeneration of the articular cartilage extracellular matrix.

## Claims

1. Use of a histone deacetylase-inhibiting compound in the production of a medicament for inhibiting articular cartilage extracellular matrix degradation.

2. The use according to claim 1, wherein the histone deacetylase-inhibiting compound is selected from FK228, a depsipeptide compound represented by the following formula (I), a depsipeptide compound represented by the following general formula (II), a depsipeptide compound represented by the following general formula (IIa), MS-27-275, Trichostatin A, NVP-LAQ824, SAHA, Apicidin, Phenylbutyrate, Valproic acid, Pivaloyloxylmethyl butyrate, CI-994, Depudecin, Trapoxin, CHAP, and butyric acid; 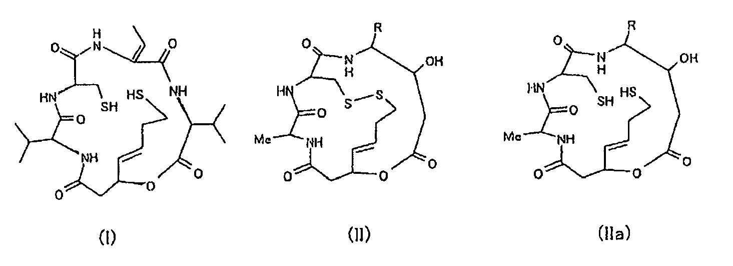 wherein R represents an isopropyl group, a sec-butyl group, or an isobutyl group.

3. The use according to claim 1, wherein the histone deacetylase-inhibiting compound is selected from FK228, the depsipeptide compound represented by the formula (I) described in claim 2, the depsipeptide compound represented by the general formula (II) described in claim 2, a depsipeptide compound represented by the general formula (IIa) described in claim 2, MS-27-275, Trichostatin A, NVP-LAQ824, SAHA, Apicidin, Phenylbutyrate, and Valproic acid.

4. The use according to claim 1, wherein the histone deacetylase-inhibiting compound is a compound whose histone deacetylase inhibitory activity IC₅₀ value) measured according to a method described in "Yoshida *et al.*, *Journal of Biological Chemistry,* 1990, Vol. 265, p17174-17179" is a concentration of 100 µm or less.

5. Use of a histone deacetylase-inhibiting compound in the manufacture of an agent for the prevention or treatment of articular cartilage extracellular matrix degradation in arthrosteitis.

6. Use of a histone deacetylase-inhibiting compound in the manufacture of an agent for the prevention or treatment of articular cartilage extracellular matrix degradation in rheumatic arthritis.

7. Use of a histone deacetylase-inhibiting compound in the manufacture of an agent for the prevention or treatment of articular cartilage extracellular matrix degradation in osteoarthritis.

## Patentansprüche

1. Verwendung einer Histon-Deacetylase-Inhibitionsverbindung bei der Herstellung eines Medikaments zum Inhibieren eines Abbaus extrazellulärer Gelenkknorpelmatrix.

2. Verwendung nach Anspruch 1, wobei die Histon-Deacetylase-Inhibitionsverbindung ausgewählt ist aus FK228, einer Depsipeptidverbindung, die durch die folgende Formel (I) repräsentiert ist, einer Depsipeptidverbindung, die durch die folgende allgemeine Formel (II) repräsentiert ist, einer Depsipeptidverbindung, die durch die folgende allgemeine Formel (IIa) repräsentiert ist, MS-27-275, Trichostatin A, NVP-LAQ824, SAHA, Apicidin, Phenylbutyrat, Val.proinsäure, Pivaloyloxylmethylbutyrat, CI-994, Depudecin, Trapoxin, CHAP und Buttersäure; 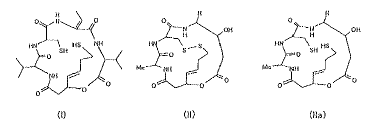 wobei R eine Isopropylgruppe, eine sec-Butylgruppe oder eine Isobutylgruppe repräsentiert.

3. Verwendung nach Anspruch 1, wobei die Histon-Deacetylase-Inhibitionsverbindung ausgewählt ist aus FK228, der durch die Formel (I) repräsentierten und in Anspruch 2 beschriebenen Depsipeptidverbindung, der durch die allgemeine Formel (II) repräsentierten und in Anspruch 2 beschriebenen Depsipeptidverbindung, einer durch die allgemeine Formel (IIa) repräsentierten und in Anspruch 2 beschriebenen Depsipeptidverbindung, MS-27-275, Trichostatin A, NVP-LAQ824, SAHA, Apicidin, Phenylbutyrat und Valproinsäure.

4. Verwendung nach Anspruch 1, wobei die Histon-Deacetylase-Inhibitionsverbindung eine Verbindung ist, deren Histon-Deacetylase-Inhibitionsaktivität (IC₅₀-Wert), gemessen mit einem in "Yoshida *et al., Journal of Biological Chemistry,* 1990, Bd. 265, S. 17174-17179" beschriebenen Verfahren, eine Konzentration von 100 µm oder weniger ist.

5. Verwendung einer Histon-Deacetylase-Inhibitionsverbindung bei der Herstellung eines Agens zur Verhütung oder Behandlung eines Abbaus von extrazellulärer Gelenkknorpelmatrix bei Arthrosteitis.

6. Verwendung einer Histon-Deacetylase-Inhibitionsverbindung bei der Herstellung eines Agens zur Verhütung oder Behandlung eines Abbaus von extrazellulärer Gelenkknorpelmatrix bei rheumatischer Arthritis.

7. Verwendung einer Histon-Deacetylase-Inhibitionsverbindung bei der Herstellung eines Agens zur Verhütung oder Behandlung eines Abbaus von extrazellulärer Gelenkknorpelmatrix bei Osteoarthritis.

## Revendications

1. Utilisation d'un composé inhibiteur d'histone-désacétylase dans la production d'un médicament pour inhiber la dégradation de la matrice extracellulaire du cartilage articulaire.

2. L'utilisation selon la revendication 1, dans laquelle le composé inhibiteur d'histone-désacétylase est sélectionné parmi FK228, un composé depsipeptide représente par la formule suivante (I), un composé depsipeptide représenté par la formule générale suivante (II), un composé depsipeptide représente par la formule générale suivante (IIa), MS-27-275, trichostatine A, NVP-LAQ824, SAHA, apicidine, phénylbutyrate, acide valproïque, butyrate de pivaloyloxyméthyle, C1-994, depudecine, trapoxine, CHAP et acide butyrique, 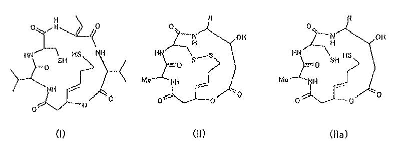 où R représente un groupe isopropyle, un groupe sec-butyle ou un groupe isobutyle.

3. L'utilisation selon la revendication 1, dans laquelle le composé inhibiteur d'histone-désacétylase est sélectionné parmi FK228, le composé depsipeptide représenté par la formule (1) décrite dans la revendication 2, le composé depsipeptide représenté par la formule générale (II) décrite dans la revendication 2, un composé depsipeptide représenté par la formule générale (IIa) décrite dans la revendication 2, MS-27-275, la trichostatine A, NVP-LAQ824, SAHA, l'apicidine, le phénylbutyrate et l'acide valproïque.

4. L'utilisation selon la revendication 1, dans laquelle le composé inhibiteur d'histone-désacétylasc est un composé dont l'activité inhibitrice d'histone-désacétylase (valeur de CI₅₀) mesurée selon une méthode décrite dans "Yoshida *et al*., .Journal of Biological Chemistry, 1990, volume 265, p. 17174-17179", est une concentration de 100 µm ou moins.

5. Utilisation d'un composé inhibiteur d'histone-désacétylase dans la fabrication d'un agent pour la prévention ou le traitement de la dégradation de la matrice extracellulaire du cartilage articulaire dans l'arthrostéite.

6. Utilisation d'un composé inhibiteur d'histone-désacétylase dans la fabrication d'un agent pour la prévention ou le traitement de la dégradation de la matrice extracellulaire du cartilage articulaire dans l'arthrite rhumatismale.

7. Utilisation d'un composé inhibiteur d'histone-désacétylase dans la fabrication d'un agent pour la prévention ou le traitement de la dégradation de la matrice extracellulaire du cartilage articulaire dans l'ostéoarthrite.
